(19) Europäisches Patentamt / European Patent Office / Office européen des brevets

(11) **EP 3 903 931 A1**

(12) **EUROPEAN PATENT APPLICATION**
published in accordance with Art. 153(4) EPC

(43) Date of publication:
**03.11.2021 Bulletin 2021/44**

(21) Application number: **19904505.5**

(22) Date of filing: **03.12.2019**

(51) Int Cl.:
**B01J 23/889** (2006.01)  **B01J 23/34** (2006.01)
**B01J 23/83** (2006.01)  **C07B 61/00** (2006.01)
**C07C 5/333** (2006.01)  **C07C 11/04** (2006.01)

(86) International application number:
**PCT/JP2019/047229**

(87) International publication number:
**WO 2020/137382 (02.07.2020 Gazette 2020/27)**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(30) Priority:  **27.12.2018  JP 2018246155
06.02.2019  JP 2019019925**

(71) Applicant: **Kubota Corporation
Osaka 556-8601 (JP)**

(72) Inventors:
• **MAEDA, Shun
Hirakata-shi, Osaka 573-8573 (JP)**
• **HASHIMOTO, Kunihide
Hirakata-shi, Osaka 573-8573 (JP)**
• **SEKINE, Yasushi
Tokyo 169-8050 (JP)**

(74) Representative: **Graf von Stosch
Patentanwaltsgesellschaft mbH
Prinzregentenstraße 22
80538 München (DE)**

(54) **DEHYDROGENATION CATALYST**

(57)    Provided is a dehydrogenating catalyst that is capable of preventing or reducing coking and improving the yield of an olefin in a pyrolysis reaction of a hydrocarbon raw material. A dehydrogenating catalyst (4A) for production of an olefin contains, as a catalyst component, at least one of La and Ce, wherein, when the dehydrogenating catalyst (4A) does not contain Ce, the dehydrogenating catalyst (4A) contains at least one element selected from the group consisting of Ba, Fe, and Mn, or wherein, when the dehydrogenating catalyst (4A) contains Ce, the dehydrogenating catalyst (4A) contains at least one of Fe and Mn.

FIG. 1

**Description**

Technical Field

The present invention relates to a dehydrogenating catalyst.

Background Art

Olefins such as ethylene and propylene are used to manufacture chemical synthetic products for various purposes of use in industries. An olefin is produced by supplying a petroleum-derived hydrocarbon such as ethane or naphtha into a pyrolysis tube (cracking tube), and pyrolyzing the hydrocarbon in a gas phase by heating at 700°C to 900°C. In the production method, a large amount of energy is required to achieve high temperature. Moreover, the process of pyrolysis of a hydrocarbon as a raw material has various problems such as deposition of carbon (coke) (such a deposition is "coking") on an inner surface of the pyrolysis tube and a carburization phenomenon that occurs on the inner surface of the pyrolysis tube. Under the circumstances, development of a high-performance dehydrogenating catalyst that can solve those problems is demanded.

For example, Patent Literature 1 discloses a perovskite-type oxide which reduces coking on the inner surface of a pyrolysis tube.

Patent Literature 2 discloses a dehydrogenating catalyst that contains, as a catalyst component, at least one selected from the group consisting of oxides of metal elements in Group 2B of the periodic table, oxides of metal elements in Group 3B of the periodic table, and oxides of metal elements in Group 4B of the periodic table.

Citation List

[Patent Literature]

[Patent Literature 1]
Specification of US Patent No. 9499747
[Patent Literature 2]
Japanese Patent Application Publication, Tokukai, No. 2017-209661

Summary of Invention

Technical Problem

However, the oxide disclosed in Patent Literature 1 and the dehydrogenating catalyst disclosed in Patent Literature 2 do not achieve a sufficiently high yield of an olefin, and therefore there is a demand for development of a dehydrogenating catalyst with higher performance.

An aspect of the present invention was made in view of the problems, and its object is to provide a dehydrogenating catalyst that is capable of preventing or reducing coking and improving the yield of an olefin in a pyrolysis reaction of a hydrocarbon raw material.

Solution to Problem

In order to attain the above objet, a dehydrogenating catalyst in accordance with an aspect of the present invention is a dehydrogenating catalyst for production of an olefin, containing, as a catalyst component, at least one of the following composite oxide and the following mixture:

a composite oxide that contains at least one of La and Ce, wherein, when the composite oxide does not contain Ce, the composite oxide contains at least one element selected from the group consisting of Ba, Fe, and Mn or wherein, when the composite oxide contains Ce, the composite oxide contains at least one of Fe and Mn and does not contain Ba; and

a mixture that contains a first oxide and a second oxide, the first oxide containing at least one of La and Ce, wherein, when the first oxide does not contain Ce, the second oxide contains at least one element selected from the group consisting of Ba, Fe, and Mn or wherein, when the first oxide contains Ce, the second oxide contains at least one of Fe and Mn.

Advantageous Effects of Invention

An aspect of the present invention brings about the effect of providing a dehydrogenating catalyst that is capable of preventing or reducing coking and improving the yield of an olefin in a pyrolysis reaction of a hydrocarbon raw material.

Brief Description of Drawings

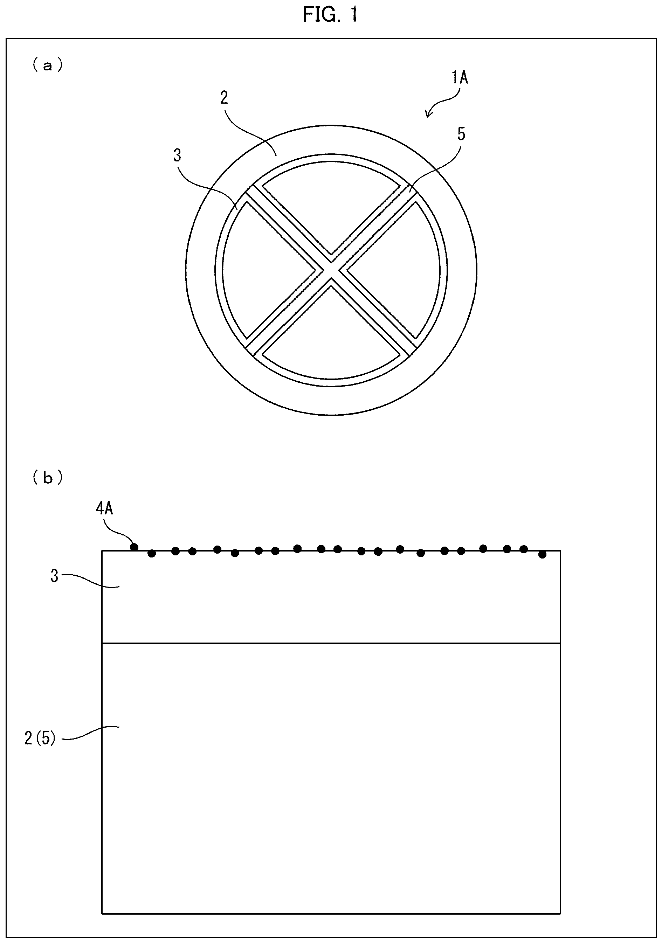

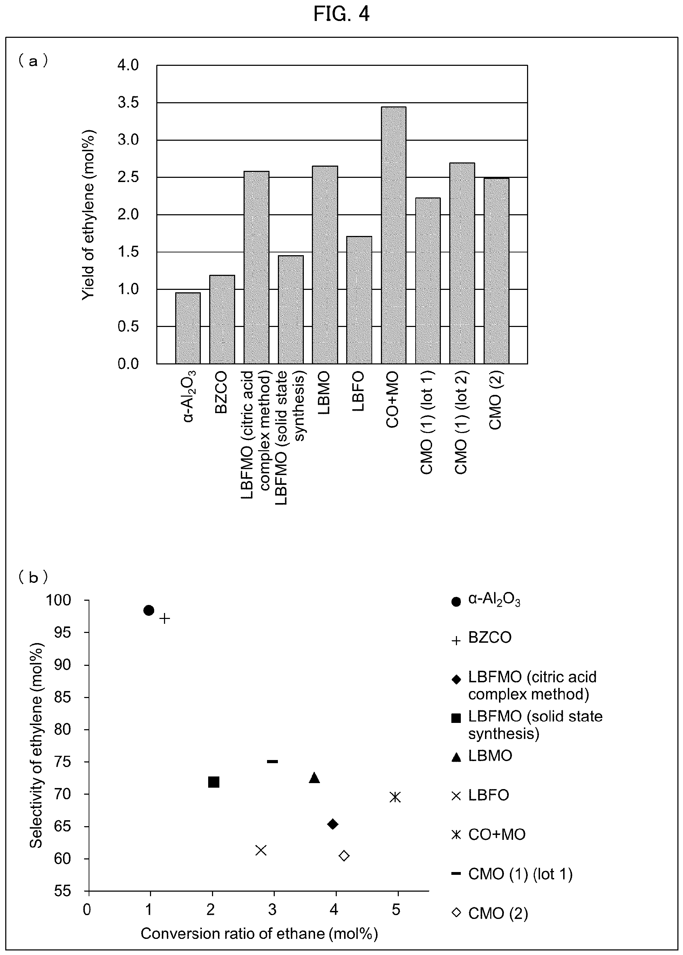

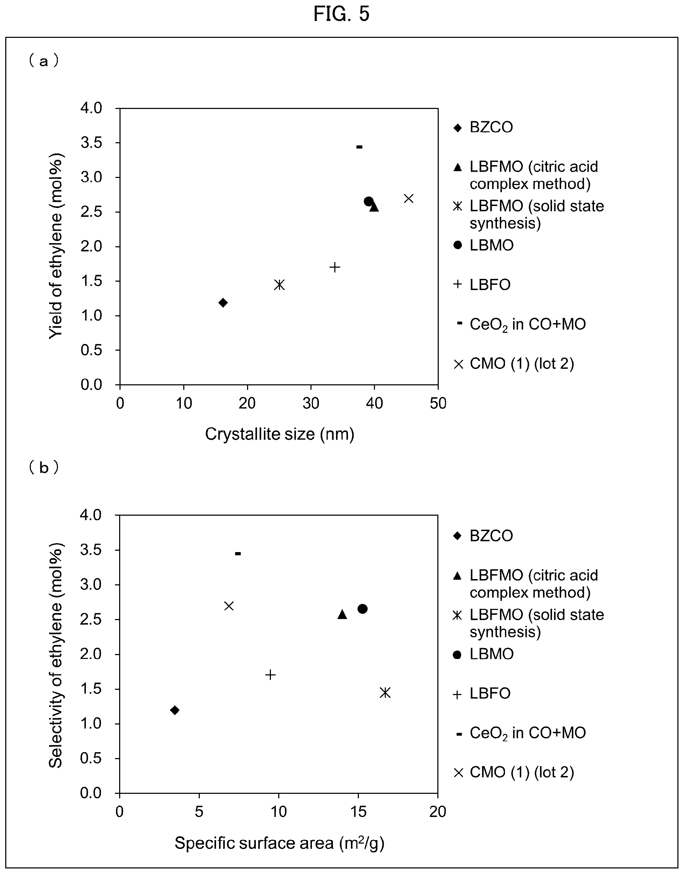

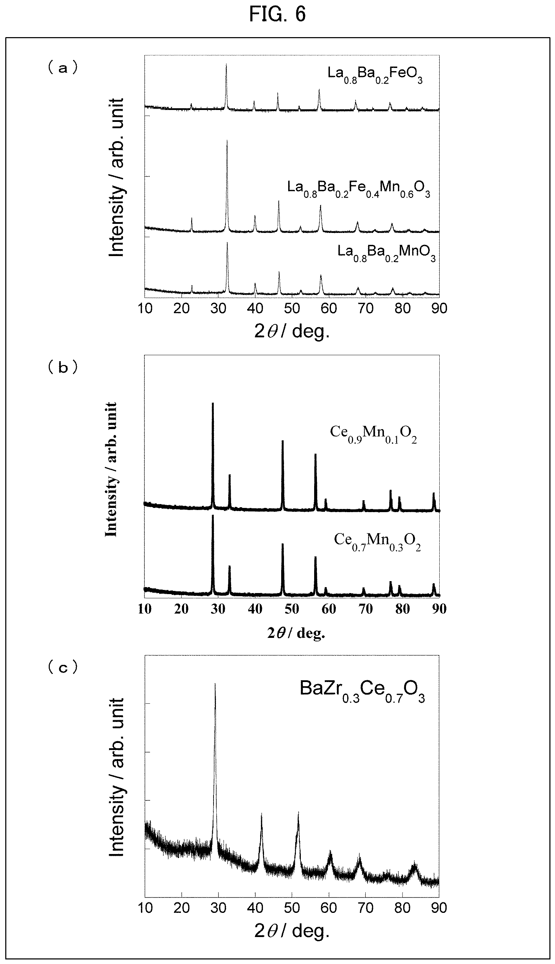

Fig. 1 illustrates a configuration of a pyrolysis tube for production of an olefin in accordance with Embodiment 1 of the present invention, in which (a) of Fig. 1 is a cross-sectional view schematically illustrating the pyrolysis tube for production of an olefin, and (b) of Fig. 1 is an enlarged view illustrating an inner surface of the pyrolysis tube for production of an olefin illustrated in (a) of Fig. 1.

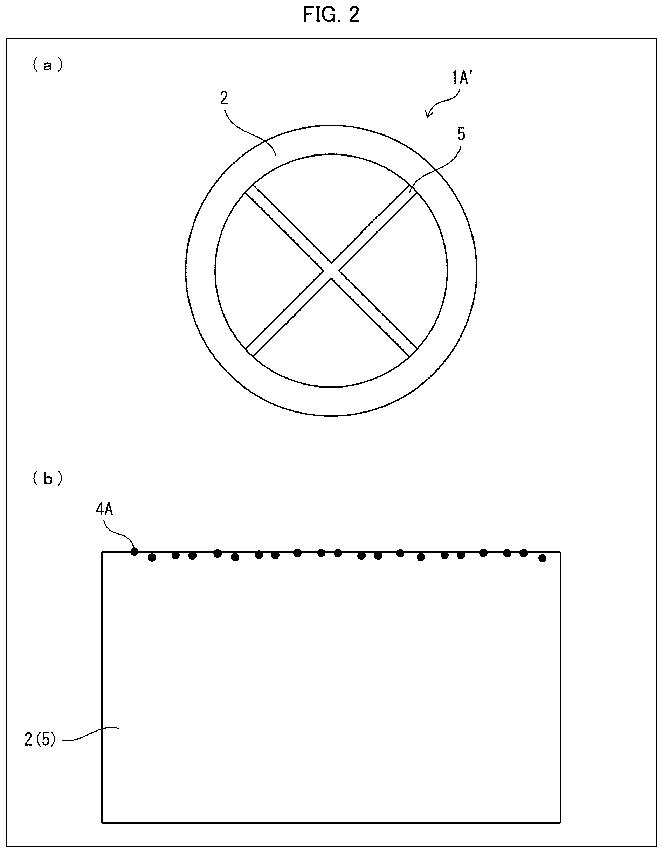

Fig. 2 illustrates a configuration of a pyrolysis tube for production of an olefin which is a modification example of the above pyrolysis tube for production of an olefin, in which (a) of Fig. 2 is a cross-sectional view schematically illustrating the pyrolysis tube for production of an olefin, and (b) of Fig. 2 is an enlarged view illustrating an inner surface of the pyrolysis tube for production of an olefin illustrated in (a) of Fig. 2.

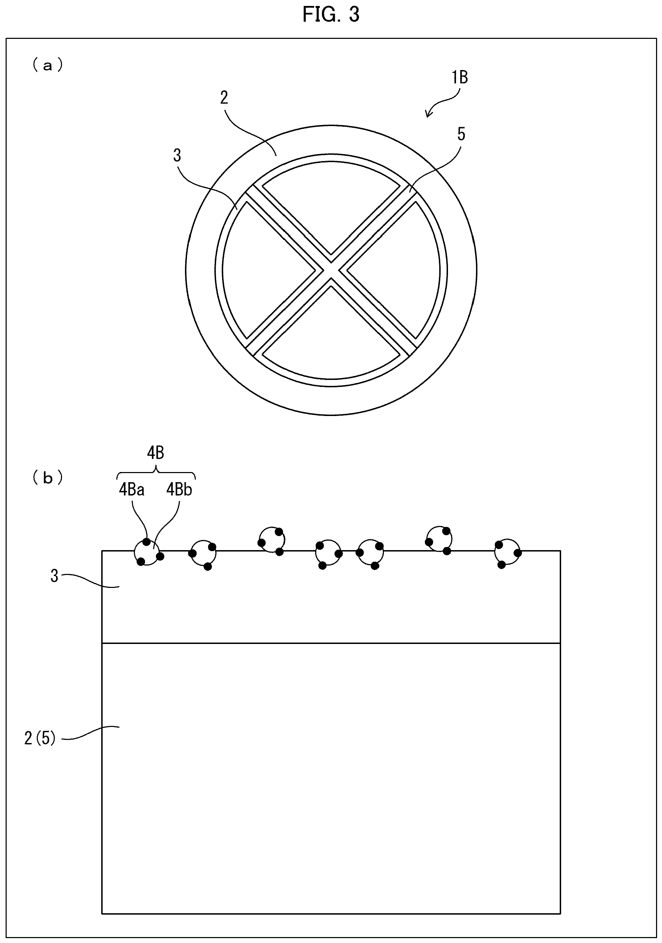

Fig. 3 illustrates a configuration of a pyrolysis tube for production of an olefin in accordance with Embodiment 2 of the present invention, in which (a) of Fig. 3 is a cross-sectional view schematically illustrating the pyrolysis tube for production of an olefin, and (b) of Fig. 3 is an enlarged view illustrating an inner surface of the pyrolysis tube for production of an olefin illustrated in (a) of Fig. 3.
(a) of Fig. 4 is a chart showing the yield of ethylene obtained in an experiment of pyrolysis of ethane which was carried out with use of dehydrogenating catalysts as Catalyst Examples and a Comparative Example and powdery $\alpha$-$Al_2O_3$. (b) of Fig. 4 is a chart showing the selectivity of ethylene versus the conversion ratio of ethane obtained in the experiment of pyrolysis of ethane which was carried out with use of dehydrogenating catalysts as Catalyst Examples and a Comparative Example and powdery $\alpha$-$Al_2O_3$.
(a) of Fig. 5 is a chart showing the yield of ethylene versus crystallite size obtained in an experiment of pyrolysis of ethane which was carried out with use of dehydrogenating catalysts as Catalyst Examples and a Comparative Example. (b) of Fig. 5 is a chart showing the yield of ethylene versus specific surface area obtained in the experiment of pyrolysis of ethane which was carried out with use of dehydrogenating catalysts as Catalyst Examples and a Comparative Example.
(a) and (b) of Fig. 6 show the results of an X-ray diffraction analysis which was carried out with respect to dehydrogenating catalysts of Catalyst Examples. (c) of Fig. 6 shows the results of an X-ray diffraction analysis which was carried out with respect to a dehydrogenating catalyst of a Comparative Example.

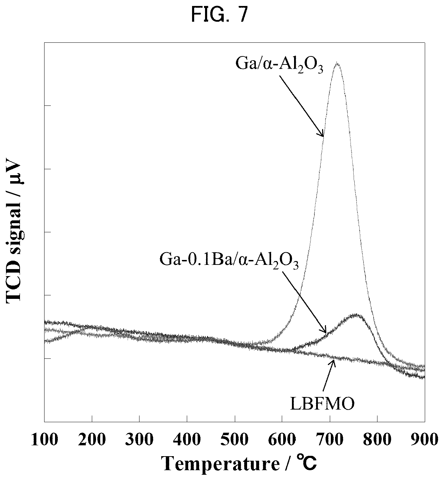

Fig. 7 is a chart showing the results of an experiment to evaluate the amount of carbon deposition which was carried out with use of dehydrogenating catalysts as a Catalyst Example and Comparative Examples.

Description of Embodiments

Embodiment 1

The following description will discuss details of a pyrolysis tube 1A for production of an olefin in accordance with Embodiment 1 of the present invention with reference to Fig. 1. Fig. 1 illustrates a configuration of the pyrolysis tube 1A in accordance with Embodiment 1, in which (a) of Fig. 1 is a cross-sectional view schematically illustrating the pyrolysis tube 1A, and (b) of Fig. 1 is an enlarged view illustrating an inner surface of the pyrolysis tube 1A illustrated in (a) of Fig. 1.
As illustrated in (a) and (b) of Fig. 1, the pyrolysis tube 1A in accordance with Embodiment 1 includes: a tubular base material 2 made of a heat resistant metal material; a plate-shaped member (insert material) 5 made of a heat resistant metal material; an alumina layer 3 which is a metal oxide layer containing $Al_2O_3$ and which is provided on an inner surface of the tubular base material 2 and on surfaces of the plate-shaped member (insert material) 5; and particles of a dehydrogenating catalyst 4A which are supported on a surface of the alumina layer 3. In the present application, the metal oxide layer containing $Al_2O_3$ is referred to as "alumina layer". With the configuration, a dehydrogenation catalytic reaction is added to a pyrolysis reaction; therefore, the pyrolysis tube 1A of an aspect of the present invention can improve the yield of an olefin obtained from a hydrocarbon raw material such as ethane or naphtha. The following description will discuss details of the base material 2, the plate-shaped member 5, the alumina layer 3, and the dehydrogenating catalyst 4A which are included in the pyrolysis tube 1A.

(Base material 2 and plate-shaped member 5)

The base material 2 in accordance with Embodiment 1 is a casting made of a heat resistant metal material. The base material 2 has the alumina layer 3 formed on the surface thereof. The plate-shaped member 5 in accordance with Embodiment 1 is provided in the space defined by the base material 2, and is a casting made of a heat resistant metal material or a stainless steel sheet. The plate-shaped member 5 has the alumina layer 3 formed on the surfaces thereof. Note that, although the pyrolysis tube 1A includes the plate-shaped member 5 in Embodiment 1, the plate-shaped member 5 is not essential and can be omitted. The base material 2 and the plate-shaped member 5 can each be, for example, a casting obtained by casting a known heat resistant metal material, and are each preferably a casting composed of a heat resistant metal material which at least contains chromium (Cr), nickel (Ni), and aluminum (Al). The base material 2 and the plate-shaped member 5 can be produced by a known method. In Embodiment 1, the alumina layer 3 is disposed on the inner surface of the base material 2 and the surfaces of the plate-shaped member 5; however, the alumina layer 3 may be disposed only on the inner surface of the base material 2 or only on the surfaces of the plate-shaped member 5. In Embodiment 1, the dehydrogenating catalyst 4A is supported on the inner surface of the base material 2 and on the surfaces of the plate-shaped member 5; however, the dehydrogenating catalyst 4A may be supported only on the inner surface of the base material 2 or only on the surfaces of the plate-shaped member 5.

It is preferable that at least part of the inner surface of the tubular base material 2 and/or the surfaces of the plate-shaped member 5 have a recess and/or a projection. This makes it possible to improve heat transfer efficiency and possible to uniformly heat a fluid flowing through the tubular base material 2.

(Alumina layer 3)

The alumina layer 3, which is provided on the inner surface of the base material 2 and on the surfaces of the plate-shaped member 5, of an aspect of the present invention has high denseness and serves as a barrier for preventing oxygen, carbon, and nitrogen from intruding into the base material 2 and the plate-shaped member 5 from outside.

In a general pyrolysis tube for production of an olefin, no metal oxide layer is provided on the inner surface of the base material 2 or the surfaces of the plate-shaped member 5. Therefore, a hydrocarbon raw material is excessively decomposed in pyrolysis due to an effect of catalysts such as nickel (Ni), iron (Fe), and cobalt (Co) which are constituent elements of the base material 2 and the surfaces of the plate-shaped member 5, and therefore coke is generated on the inner surface of the base material 2 and the surfaces of the plate-shaped member 5. In a case where coke generated on the inner surface of the base material 2 and the surfaces of the plate-shaped member 5 accumulates, heat transfer resistance rises. This causes the following problem: that is, the temperature of an outer surface of the pyrolysis tube for production of an olefin rises to maintain the reaction temperature in the pyrolysis tube. Moreover, in a case where coke accumulates on the inner surface of the base material 2 and the surfaces of the plate-shaped member 5, a cross-sectional area of a flow channel through which gas passes becomes smaller, and this leads to an increase in pressure loss. For these reasons, in the general pyrolysis tube for production of an olefin, it has been necessary to frequently remove accumulated coke (i.e., decoking).

On the other hand, in the pyrolysis tube 1A of Embodiment 1, the alumina layer 3 is provided on the inner surface of the base material 2 and the surfaces of the plate-shaped member 5, and this makes it possible to prevent or reduce generation of coke on the inner surface of the base material 2 and the surfaces of the plate-shaped member 5. As a result, it is possible to reduce the frequency of carrying out decoking.

A method of forming the alumina layer 3 of an aspect of the present invention includes a surface treatment step and a first heat treatment step. The following description will discuss details of the surface treatment step and the first heat treatment step.

<Surface treatment step>

The surface treatment step involves carrying out a surface treatment with respect to target sites of the base material 2 and the plate-shaped member 5 which target sites are to make contact with a high temperature atmosphere when the product is used, and adjusting the surface roughness of the target site.

The surface treatment of the base material 2 and the plate-shaped member 5 can be, for example, polishing. The surface treatment can be carried out so that the surface roughness (Ra) of the target sites becomes 0.05 $\mu$m to 2.5 $\mu$m. More preferably, the surface roughness (Ra) is 0.5 $\mu$m to 2.0 $\mu$m. Moreover, by adjusting the surface roughness in the surface treatment, it is possible to concurrently remove residual stress and distortion of a heat affected zone.

<First heat treatment step>

The first heat treatment step involves applying, in an oxidizing atmosphere, a heat treatment to the base material 2 and the plate-shaped member 5 which have been subjected to the surface treatment step.

The oxidizing atmosphere indicates an oxidizing gas containing oxygen in an amount of 20 volume% or more or an oxidizing environment in which steam and $CO_2$ are mixed. The heat treatment is carried out at a temperature of 900°C or higher, preferably 1000°C or higher, and a heating time is 1 hour or longer.

By sequentially carrying out the surface treatment step and the first heat treatment step with respect to the base material 2 and the plate-shaped member 5 as described above, it is possible to obtain a pyrolysis tube for production of an olefin in which the alumina layer 3 is stably provided on the inner surface of the base material 2 and the surfaces of the plate-shaped member 5.

The thickness of the alumina layer 3 which is provided on the inner surface of the base material 2 and the surfaces of the plate-shaped member 5 is suitably 0.5 $\mu$m or more and 6 $\mu$m or less in order to effectively achieve a barrier function. In a case where the thickness of the alumina layer 3 is less than 0.5 $\mu$m, carburization resistance may decrease. In a case where the thickness of the alumina layer 3 is more than 6 $\mu$m, the alumina layer 3 may easily peel off due to the influence of a difference in thermal expansion coefficient between (i) the base material 2 and the plate-shaped member 5 and (ii) the layer.

In order to avoid such an influence, the thickness of the alumina layer 3 is more suitably 0.5 $\mu$m or more and 2.5 $\mu$m or less.

Note that, in a case where a surface of the pyrolysis tube 1A of an aspect of the present invention is investigated by SEM/EDX, chromium oxide scales are sometimes partially observed on the alumina layer 3. This is because chromium oxide scales formed in the vicinity of the surface of the base material 2 and the surfaces of the plate-shaped member 5 are forced up to the surface of the product by $Al_2O_3$. It is preferable that the chromium oxide scales less appear, and therefore the area of chromium oxide scales suitably accounts for 20% or less of the entire surface of the product so that the area of the $Al_2O_3$ accounts for 80% or more of the entire surface of the product.

(Dehydrogenating catalyst 4A)

The dehydrogenating catalyst 4A is a dehydrogenating catalyst for production of an olefin. The dehydrogenating catalyst 4A is a catalyst for improving the yield of an olefin in a pyrolysis reaction (specifically, a reaction by which a hydrocarbon raw material such as naphtha or ethane is pyrolyzed into an olefin) carried out with use of the pyrolysis tube 1A. The dehydrogenating catalyst 4A is supported on a surface of the alumina layer 3.

The dehydrogenating catalyst 4A contains, as a catalyst component, at least one of the following composite oxide and the following mixture: a composite oxide that contains at least one of La and Ce, wherein, when the composite oxide does not contain Ce, the composite oxide contains at least one element selected from the group consisting of Ba, Fe, and Mn or wherein, when the composite oxide contains Ce, the composite oxide contains at least one of Fe and Mn and does not contain Ba; and a mixture that contains a first oxide and a second oxide, the first oxide containing at least one of La and Ce, wherein, when the first oxide does not contain Ce, the second oxide contains at least one element selected from the group consisting of Ba, Fe, and Mn or wherein, when the first oxide contains Ce, the second oxide contains at least one of Fe and Mn. This makes it possible to improve the conversion ratio of a hydrocarbon raw material. Even if the conversion ratio of the hydrocarbon raw material is improved only by several percentages by mole (e.g., about 1 to 2 mol%), such an improvement will result in a large improvement in yield of an olefin in an industrial-scale production. Therefore, an improvement in the conversion ratio of a hydrocarbon raw material more significantly affects the yield of an olefin than an improvement in olefin selectivity. Since it is possible to improve the conversion ratio of a hydrocarbon raw material, it is possible to improve the yield of an olefin in a pyrolysis reaction of the hydrocarbon raw material. Furthermore, the pyrolysis reaction of the hydrocarbon raw material is carried out at a temperature of, for example, not lower than 700°C, and therefore coking resulting from excessive decomposition is generally likely to occur; however, since the dehydrogenating catalyst 4A has the foregoing configuration, it is possible to prevent or reduce the coking.

The composite oxide can be, for example, an oxide composed of La, Ba, Fe, Mn, and O, an oxide compose of La, Ba, Mn, and O, an oxide composed of La, Ba, Fe, and O, an oxide composed of Ce, Mn, and O, an oxide composed of La, Ce, Mn, and O ($La_aCe_bMn_cO_d$), or the like.

The first oxide can be, for example, $CeO_2$, $La_2O_3$ or the like. The second oxide can be, for example, $Mn_2O_3$, $LaMnO_3$, or the like. The mixture can be, for example, a mixture of $CeO_2$ and $Mn_2O_3$, a mixture of $La_2O_3$ and $Mn_2O_3$, or the like.

The composite oxide or the first oxide has a crystallite size of preferably 20 nm to 75 nm, more preferably 20 nm to 50 nm, even more preferably 20 nm to 40 nm. This makes it possible to improve the yield of an olefin in a pyrolysis reaction by which a hydrocarbon raw material is pyrolyzed into the olefin. The crystallite size is measured by X-ray diffractometry.

The composite oxide is preferably a perovskite-type oxide. The perovskite-type oxide is a composite oxide which has a perovskite structure represented by $ABO_3$. In a case where the composite oxide does not contain Ce, the A sites contain at least one of La and Ba and the B sites contain at least one of Mn and Fe. In a case where the composite oxide contains Ce, the A sites contain Ce and the B sites contain at least one of Mn and Fe. In a case where the composite oxide contains La and Ce, the A sites contain La and Ce and the B sites contain at least one of Mn and Fe.

The perovskite structure is distorted in its crystal structure due to differences in size between the elements in the A sites and B sites. Oxygen for the crystal lattice (lattice oxygen) more easily enters and goes out of the perovskite structure than a structure without distortion.

Constituent elements in a perovskite structure can be replaced while maintaining the perovskite structure, provided that the tolerance factor of the perovskite structure is within a certain range. This makes it possible to impart properties of various elements to the perovskite structure. Furthermore, a replacement of constituent elements results in a change in size of constituent elements. This leads to a change in the degree of distortion of the crystal structure, resulting in a change in migration ability of lattice oxygen.

A perovskite structure is formed in a manner that depends on the sizes of AO layers and $BO_2$ layers stacked alternately on top of each other. The tolerance factor is an indicator that quantifies this, and is represented by the following equation (1):

$$(1) \qquad t = r_A / r_O / \sqrt{2}(r_B + r_O),$$

where $r_A$, $r_B$, and ro represent the ion radii of A, B, and O ions, respectively. A perovskite-type oxide appears when t is about 1.05 to 0.90, and an ideal perovskite structure is realized when t is 1.

In a perovskite-type oxide, lattice oxygen easily moves; therefore, the perovskite-type oxide has redox ability, and dehydrogenation (oxidative dehydrogenation) takes place via oxygen. Oxidative dehydrogenation is generally higher in reactivity than simple dehydrogenation.

Furthermore, since lattice oxygen easily moves in a perovskite-type oxide, the lattice oxygen reacts with coke (C) attached on the surface of the catalyst to form a gas such as carbon monoxide (CO) and carbon dioxide ($CO_2$). This makes it possible to prevent the deposition of coke (accumulation of coke) or reduce the amount of deposited coke (the amount of accumulated coke).

The specific surface area (BET specific surface area) of the dehydrogenating catalyst 4A is preferably 5 m$^2$/g to 80m$^2$/g, more preferably 5 m$^2$/g to 40 m$^2$/g, even more preferably 5 m$^2$/g to 20 m$^2$/g. This makes it possible to improve the yield of an olefin in a pyrolysis reaction by which a hydrocarbon raw material is pyrolyzed into the olefin. Furthermore, although the pyrolysis reaction generally proceeds faster when the specific surface area is larger, too large a specific surface area is likely to result in deposition of coke. When the specific surface area is not less than 5 m$^2$/g, the pyrolysis reaction proceeds fast. When the specific surface area is not more than 80 m$^2$/g, it is possible to eliminate or reduce the likelihood that the amount of coke will be too large. Note that, in a case where the composite oxide or the mixture is not supported on a carrier like the dehydrogenating catalyst 4A of Embodiment 1, the above-mentioned specific surface area is the specific surface area of the composite oxide or of the mixture (dehydrogenating catalyst 4A). In a case where the composite oxide or the mixture is supported on a carrier like a dehydrogenating catalyst 4B of Embodiment 2 (describe later), the above-mentioned specific surface area is the specific surface area of a dehydrogenating catalyst 4B which is made up of a catalyst component 4Ba and a carrier 4Bb on which the catalyst component 4Ba is supported.

<Method of producing dehydrogenating catalyst 4A>

The dehydrogenating catalyst 4A is produced preferably by a citric acid complex method or a solid state synthesis.

(Citric acid complex method)

The citric acid complex method includes a mixing/stirring step, a drying step, a calcining step, and a final firing step.

The mixing/stirring step involves mixing salts (e.g., nitrate, acetate) containing elements of the dehydrogenating catalyst 4A, citric acid monohydrate, ethylene glycol, and distilled water to obtain a liquid mixture. The salts are weighed out so that the La, Ce, Ba, Fe, and Mn have a desired molar ratio. The citric acid monohydrate is added so that the molar quantity of the citric acid monohydrate is preferably 3 to 4 times the total molar quantity of La, Ce, Ba, Fe, and Mn which are contained in the salts. The ethylene glycol is added so that the molar quantity of the ethylene glycol is preferably 3 to 4 times the total molar quantity of La, Ce, Ba, Fe, and Mn which are contained in the salts. The distilled water is added so that the molar quantity of the distilled water is preferably 1200 to 1600 times the total molar quantity of La, Ce, Ba, Fe, and Mn which are contained in the salts. It is preferable that the liquid mixture be stirred at 60°C to 70°C for 10 hours to 17 hours.

The drying step involves drying the liquid mixture to obtain powder. For example, it is only necessary to heat and dry the liquid mixture while stirring the liquid mixture on a hot plate.

The calcining step involves calcining the powder to obtain a calcined product. The calcining step is carried out preferably in air or in oxygen, the temperature at which the calcining step is carried out (hereinafter "calcination temperature") is preferably 400°C to 450°C, and the time for which the temperature is maintained (hereinafter "temperature maintenance time") is preferably 2 hours to 3 hours. The calcination temperature and the temperature maintenance time may be adjusted appropriately within the above-stated ranges depending on the amount of a catalyst to be prepared.

The final firing step involves finally firing the calcined product to obtain an oxide. The final firing step is carried out preferably in air or in oxygen, the temperature at which the final firing step is carried out (hereinafter "final firing temperature") is preferably 850°C to 900°C, and the time for which the temperature is maintained (hereinafter "temperature maintenance time") is preferably 8 hours to 12 hours. The final firing temperature and the temperature maintenance time may be adjusted appropriately within the above-stated ranges depending on the amount of a catalyst to be prepared.

(Solid state synthesis)

The solid state synthesis includes a pulverizing/mixing step, a drying step, and a firing step

The pulverizing/mixing step involves: mixing compounds (e.g., oxide, carbonate compound) containing elements of the dehydrogenating catalyst 4A to obtain a mixture; and pulverizing the mixture and mixing the particles of the mixture to obtain pulverized, mixed powder. The compounds are mixed so that La, Ce, Ba, Fe, and Mn have a desired molar ratio. The compounds may be mixed and pulverized with use of, for example, a wet-type bead mill.

The drying step involves drying the pulverized, mixed powder to obtain a dried product.

The firing step involves firing the dried product to obtain an oxide. The firing step is carried out preferably in air or in oxygen, the temperature at which the firing step is carried out (hereinafter "firing temperature") is preferably 500°C to 1300°C, and the time for which the temperature is maintained (hereinafter "temperature maintenance time") is preferably 1 hour to 10 hours. The firing temperature and the temperature maintenance time may be adjusted appropriately within the above-stated ranges depending on the amount of a catalyst to be prepared.

<Method for causing dehydrogenating catalyst 4A to be supported>

The following description will discuss a method for causing the dehydrogenating catalyst 4A to be supported on the alumina layer 3. The method for causing the dehydrogenating catalyst 4A to be supported on the alumina layer 3 includes an applying step and a second heat treatment step. The following description will discuss details of the applying step and the second heat treatment step.

(a) Applying step

The applying step involves applying a slurry containing the dehydrogenating catalyst 4A prepared in advance to the surface of the alumina layer 3 which has been formed by the surface treatment step and the first heat treatment step.

(b) Second heat treatment step

The second heat treatment step involves heating the base material 2 and the plate-shaped member 5 on which the slurry has been applied to the alumina layer 3 in the applying step.

The heat treatment in the second heat treatment step is carried out in air or in an acidic atmosphere. A heat treatment temperature in the second heat treatment step is within a range from 500°C to 900°C, and a heat treatment time is 1 hour to 6 hours.

Carrying out the second heat treatment step under the above heat treatment conditions makes it possible to allow the dehydrogenating catalyst 4A to be supported on the alumina layer 3.

Note that the dehydrogenating catalyst 4A can be supported on the alumina layer 3 at an appropriate concentration (amount) by adjusting the concentration of the slurry that is applied in the applying step.

As such, the pyrolysis tube 1A in accordance with Embodiment 1 includes: the tubular base material 2 made of a heat resistant metal material; the plate-shaped member 5 made of a heat resistant metal material; the alumina layer 3 which is provided on the inner surface of the tubular base material 2 and/or the surfaces of the plate-shaped member 5; and the dehydrogenating catalyst 4A which is supported on the surface of the alumina layer 3.

According to the configuration, the pyrolysis tube 1A of an aspect of the present invention is configured such that the alumina layer 3 is provided on the inner surface of the base material 2 and the surfaces of the plate-shaped member 5. This makes it possible to prevent or reduce generation of coke on the surface of the alumina layer 3 (base material 2 and plate-shaped member 5). Further, the dehydrogenating catalyst 4A is supported on the surface of the alumina layer 3. With the configuration, when the dehydrogenating catalyst 4A functions as a dehydrogenating catalyst in pyrolysis carried out with use of the pyrolysis tube 1A, for example, it is possible to generate ethylene from ethane by dehydrogenation. As a result, it is possible to improve the yield of an olefin obtained from pyrolysis of a hydrocarbon raw material such as ethane or naphtha.

Moreover, in Embodiment 1, the dehydrogenating catalyst 4A is supported on the alumina layer 3 by carrying out the applying step and the second heat treatment step with respect to the alumina layer 3 which has been formed on the inner surface of the base material 2 and the surfaces of the plate-shaped member 5 by the surface treatment step and the first heat treatment step. Note, however, that the pyrolysis tube for production of an olefin of the present invention is not limited to this. For example, the applying step and a heat treatment step may be carried out after the surface treatment step. In this case, in the heat treatment step, the alumina layer 3 is formed on the inner surface of the base material 2 and the surfaces of the plate-shaped member 5 and also the dehydrogenating catalyst 4A is supported on the alumina layer 3. This makes it possible to form the alumina layer 3 on the inner surface of the base material 2 and the surfaces of the plate-shaped member 5 and also to cause the dehydrogenating catalyst 4A to be supported on the alumina layer 3 by carrying out the heat treatment step only once.

In Embodiment 1, the following configuration is employed: the dehydrogenating catalyst 4A is supported on the surface of the alumina layer 3 which is provided on the inner surface of the base material 2 and the surfaces of the plate-shaped member 5. Note, however, that the pyrolysis tube 1A of the present invention is not limited to this. That is, the pyrolysis tube for production of an olefin in accordance with an aspect of the present invention can employ a configuration in which the dehydrogenating catalyst 4A is supported on a surface of a metal oxide layer (e.g., $Cr_2O_3$, $MnCr_2O_4$, or the like) which is not $Al_2O_3$, which has a barrier function, and which can support the dehydrogenating catalyst 4A.

<Modification example>

The following description will discuss a pyrolysis tube 1A', which is a modification example of the pyrolysis tube 1A in Embodiment 1, with reference to Fig. 2. Fig. 2 illustrates a configuration of the pyrolysis tube 1A', in which (a) of Fig. 2 is a cross-sectional view schematically illustrating the pyrolysis tube 1A', and (b) of Fig. 2 is an enlarged view illustrating an inner surface of the pyrolysis tube 1A' illustrated in (a) of Fig. 2.

The pyrolysis tube 1A in accordance with Embodiment 1 is configured such that the alumina layer 3 which is a metal oxide layer containing $Al_2O_3$ is provided on the inner surface of the base material 2 and the surfaces of the plate-shaped member 5 and that the dehydrogenating catalyst 4A is supported on the surface of the alumina layer 3. The pyrolysis tube 1A', which is a modification example, is different from the pyrolysis tube 1A in that the dehydrogenating catalyst 4A is directly supported on the inner surface of the tubular base material 2 made of a heat resistant metal material and the surfaces of the plate-shaped member 5 made of a heat resistant metal material (see (a) and (b) of Fig. 2).

With regard to the pyrolysis tube 1A' of this modification example, a slurry which contains the dehydrogenating catalyst 4A prepared in advance is applied to the inner surface of the base material 2 and the surfaces of the plate-shaped member 5, and a heat treatment is carried out under appropriate conditions such as in air or in a nitrogen atmosphere. With this, the dehydrogenating catalyst 4A can be supported on the inner surface of the base material 2 and the surfaces of the plate-shaped member 5.

As described above, the pyrolysis tube 1A' is configured such that the dehydrogenating catalyst 4A is supported on the inner surface of the base material 2 and the surfaces of the plate-shaped member 5. With the configuration, when the dehydrogenating catalyst 4A functions as a dehydrogenating catalyst in pyrolysis carried out with use of the pyrolysis tube 1A', for example, it is possible to generate ethylene from ethane by dehydrogenation. As a result, it is possible to improve the yield of an olefin obtained from pyrolysis of a hydrocarbon raw material such as ethane or naphtha.

Embodiment 2

The following description will discuss another embodiment of the present invention. For convenience of explanation, the same reference numerals are given to constituent members which have functions identical with those described in Embodiment 1, and descriptions regarding such constituent members are omitted.

In a pyrolysis tube 1B for production of an olefin in accordance with Embodiment 2, a dehydrogenating catalyst has a configuration different from that of the dehydrogenating catalyst 4A in Embodiment 1.

(Dehydrogenating catalyst 4B)

The following description will discuss details of the pyrolysis tube 1B in accordance with Embodiment 2 of the present invention with reference to Fig. 3. Fig. 3 illustrates a configuration of the pyrolysis tube 1B in accordance with Embodiment 2, in which (a) of Fig. 3 is a cross-sectional view schematically illustrating the pyrolysis tube 1B, and (b) of Fig. 3 is an enlarged view illustrating an inner surface of the pyrolysis tube 1B illustrated in (a) of Fig. 3.

A dehydrogenating catalyst 4B for the pyrolysis tube 1B of Embodiment 2 contains a catalyst component 4Ba and a carrier 4Bb for supporting the catalyst component (see (a) and (b) of Fig. 3). Note that the catalyst component 4Ba is identical to the dehydrogenating catalyst 4A described in the "(Dehydrogenating catalyst 4A)" section.

The carrier 4Bb is a carrier for supporting the catalyst component 4Ba in the dehydrogenating catalyst 4B. The carrier 4Bb preferably has a large specific surface area in order to improve the catalytic function of the catalyst component 4Ba. Specifically, the specific surface area of the carrier 4Bb is preferably 20 $m^2$/g or more, more preferably 40 $m^2$/g or more. With the configuration, it is possible to allow particles of the catalyst component 4Ba to be highly dispersed on the carrier 4Bb. As a result, it is possible to improve the yield of an olefin in a pyrolysis reaction by which a hydrocarbon raw material is pyrolyzed into the olefin. The carrier 4Bb can be, for example, alumina ($Al_2O_3$), silica ($SiO_2$), or the like. Note that $Al_2O_3$ has the following four phases: that is, $\gamma$-$Al_2O_3$, $\delta$-$Al_2O_3$, $\theta$-$Al_2O_3$, and $\alpha$-$Al_2O_3$. For example, in a case where $\gamma$-$Al_2O_3$ is subjected to heat treatment, phase transformation occurs in the following order: ($\gamma$-$Al_2O_3$) $\rightarrow$ ($\delta$-$Al_2O_3$) $\rightarrow$ ($\theta$-$Al_2O_3$) $\rightarrow$ ($\alpha$-$Al_2O_3$) as a heat treatment temperature rises. As the phase transformation proceeds, the specific surface area becomes smaller. In particular, $\alpha$-$Al_2O_3$, whose phase is transformed at the highest temperature, has a specific surface area of 15 $m^2$/g or less. Therefore, the carrier 4Bb of the dehydrogenating catalyst 4B in accordance with Embodiment 2 preferably has a specific surface area of 20 $m^2$/g or more as described above. This means that it is preferable that the carrier 4Bb mainly contain $\gamma$-$Al_2O_3$, $\delta$-$Al_2O_3$, or $\theta$-$Al_2O_3$.

Note that, in a case where $\gamma$-$Al_2O_3$ is used as a starting material for the carrier 4Bb, the phase of $\gamma$-$Al_2O_3$ is gradually transformed by heat treatment, and therefore $Al_2O_3$ serving as the carrier 4Bb does not have a single phase except before the heat treatment and after the heat treatment at a high temperature of 1300°C or higher. That is, $\gamma$-$Al_2O_3$, $\delta$-$Al_2O_3$, $\theta$-$Al_2O_3$, and $\alpha$-$Al_2O_3$ would exist in a mixed manner. For this reason, the specific surface area of $Al_2O_3$ serving as the carrier 4Bb is the average of specific surface areas of the mixed phases of $Al_2O_3$.

The carrier 4Bb preferably forms a composite oxide or a solid solution with the catalyst component 4Ba in production of the dehydrogenating catalyst 4B. This makes it possible to inhibit aggregation of particles of the catalyst component 4Ba in the pyrolysis reaction by which a hydrocarbon raw material is pyrolyzed into an olefin. Consequently, it is possible to maintain a state in which the yield of an olefin is high for a long time, and this makes it possible to further improve the yield of the olefin. Specifically, it is preferable that at least part of the carrier 4Bb is $\theta$-$Al_2O_3$.

<Method of producing dehydrogenating catalyst 4B>

The following description will discuss a method of producing the dehydrogenating catalyst 4B. In the descriptions below, two cases of the method of producing the dehydrogenating catalyst 4B will be discussed, that is, (1) a case where $\alpha$-$Al_2O_3$ is used as a starting material for the carrier 4Bb and (2) a case where $\gamma$-$Al_2O_3$ is used as a starting material for the carrier 4Bb are described.

(1) Case where $\alpha$-$Al_2O_3$ is used as a starting material for the carrier 4Bb

The dehydrogenating catalyst 4B can be produced by causing an aqueous solution which contains the catalyst component 4Ba to adhere to $\alpha$-$Al_2O_3$ used as a starting material for the carrier 4Bb, and then carrying out heat treatment. The heat treatment is carried out in air or in oxygen, a heat treatment temperature is within a range from 500°C to 1300°C, and a heat treatment time is 1 hour to 6 hours. By carrying out the heat treatment under the above conditions, it is possible to obtain the dehydrogenating catalyst 4B in which the catalyst component 4Ba is supported on $\alpha$-$Al_2O_3$ serving as the carrier 4Bb.

(2) Case where $\gamma$-$Al_2O_3$ is used as a starting material for carrier 4Bb

The dehydrogenating catalyst 4B can be produced by causing an aqueous solution which contains the catalyst component 4Ba to adhere to $\gamma$-$Al_2O_3$ used as a starting material for the carrier 4Bb (adhering step), and then carrying out heat treatment (heat treatment step) with respect to $\gamma$-$Al_2O_3$ to which the aqueous solution has adhered. The heat treatment is carried out in air or in oxygen, a heat treatment temperature is within a range from 500°C to 1300°C, and a heat treatment time is 1 hour to 6 hours. By carrying out the heat treatment under the above conditions, it is possible to obtain the dehydrogenating catalyst 4B in which the catalyst component 4Ba is supported on $Al_2O_3$ ($\gamma$-$Al_2O_3$, $\delta$-$Al_2O_3$, $\theta$-$Al_2O_3$, or $\alpha$-$Al_2O_3$) which serves as the carrier 4Bb.

Note that the heat treatment temperature is preferably within a range from 500°C to 1100°C. This is because, in a case where the heat treatment temperature is within the range from 500°C to 1100°C, it is possible to inhibit $\gamma$-$Al_2O_3$ from being completely phase-transformed into $\alpha$-$Al_2O_3$ during the heat treatment, and this makes it possible to reduce a decrease in specific surface area of $Al_2O_3$ serving as a carrier. As a result, it is possible to allow particles of the catalyst component 4Ba to be highly dispersed on $Al_2O_3$ serving as a carrier.

The heat treatment temperature is more preferably within a range from 1000°C to 1100°C. This is because, in a case where the heat treatment temperature falls within 1000°C to 1100°C, at least part of $\gamma$-$Al_2O_3$ is phase-transformed into $\theta$-$Al_2O_3$ during heat treatment, at least part of $Al_2O_3$ is coupled with the catalyst component 4Ba in the phase transformation, and thus a composite oxide or a solid solution is formed. This makes it possible to inhibit aggregation of particles of the catalyst component 4Ba in the pyrolysis reaction by which a hydrocarbon raw material is pyrolyzed into an olefin.

The heat treatment temperature is more preferably within a range from 1000°C to 1080°C. This is because, in a case where the heat treatment temperature is within the range from 1000°C to 1100°C, it is possible to increase the proportion of $\gamma$-$Al_2O_3$ transformed to $\theta$-$Al_2O_3$ during the heat treatment.

<Method for causing dehydrogenating catalyst 4B to be supported>

The following description will discuss a method for causing the dehydrogenating catalyst 4B to be supported on the alumina layer 3. The method for causing the dehydrogenating catalyst 4B to be supported on the alumina layer 3 includes an applying step and a third heat treatment step. The following description will discuss details of the applying step and the third heat treatment step.

(a) Applying step

The applying step involves applying a slurry containing the dehydrogenating catalyst 4B to a surface of the alumina layer 3 which has been formed by the surface treatment step and the first heat treatment step which are described in Embodiment 1.

(b) Third heat treatment step

The third heat treatment step involves heating the base material 2 and the plate-shaped member 5 on which the slurry containing the dehydrogenating catalyst 4B has been applied to the alumina layer 3 by the applying step.

The heat treatment in the third heat treatment step is carried out in air or in oxygen. A heat treatment temperature in the third heat treatment step is within a range from 500°C to 900°C, and a heat treatment time is 1 hour to 6 hours.

By carrying out the third heat treatment step under the above heat treatment conditions, it is possible to cause the dehydrogenating catalyst 4B to be supported on the alumina layer 3.

Next, characteristics of the dehydrogenating catalyst 4B supported on the alumina layer 3 are described.

Note that the dehydrogenating catalyst 4B can be supported on the alumina layer 3 at an appropriate concentration (amount) by adjusting the concentration of the slurry that is applied in the applying step.

Embodiment 3

The following description will discuss a further embodiment of the present invention. For convenience of explanation, the same reference numerals are given to constituent members which have functions identical with those described in Embodiments 1 and 2, and descriptions regarding such constituent members are omitted.

A method of producing an olefin in accordance with an aspect of the present invention involves producing an olefin with use of the foregoing pyrolysis tube 1A, 1A', or 1B for production of an olefin. Examples of the olefin include ethylene and propylene. Examples of a hydrocarbon raw material include ethane and naphtha.

An olefin is produced by passing a hydrocarbon raw material through the pyrolysis tube 1A, 1A', or 1B, heating the hydrocarbon raw material to 700°C to 900°C, and pyrolyzing the hydrocarbon raw material in a gas phase.

The present invention is not limited to the embodiments, but can be altered by a skilled person in the art within the scope of the claims. The present invention also encompasses, in its technical scope, any embodiment derived by combining technical means disclosed in differing embodiments.

Examples

(i) First Working Example

The following description will discuss Working Examples of a dehydrogenating catalyst for use in a pyrolysis tube for production of an olefin in accordance with an aspect of the present invention.

<Method of producing dehydrogenating catalyst>

(Catalyst Example 1)

A dehydrogenating catalyst of Catalyst Example 1 was prepared by a citric acid complex method. Lanthanum nitrate hexahydrate ($La(NO_3)_3 \cdot 6H_2O$), barium nitrate ($Ba(NO_3)_2$), ferric nitrate nonahydrate ($Fe(NO_3)_3 \cdot 9H_2O$), and manganese nitrate hexahydrate ($Mn(NO_3)_3 \cdot 6H_2O$) were weighed out so that La, Ba, Fe, and Mn would have a molar ratio of 0.8:0.2:0.4:0.6, and used as a solute. Citric acid monohydrate and ethylene glycol each in a molar quantity of 3 times the total molar quantity of the La, Ba, Fe, and Mn contained in the solute were dissolved in distilled water in a molar quantity of 1500 times that total molar quantity, and thorough stirring was carried out to obtain a solvent. The solute was mixed into the solvent, and heated with stirring overnight at 70°C. Then, heating and drying were carried out with stirring on a hot plate to obtain powder. The powder was calcined under a condition in which the calcination temperature was 400°C and the temperature maintenance time was 2 hours, and finally fired under a condition in which the final firing temperature was 850°C and the temperature maintenance time was 10 hours. In this way, $La_{0.8}Ba_{0.2}Fe_{0.4}Mn_{0.6}O_3$ (hereinafter referred to as "LBFMO") was prepared. In the following description, the LBFMO of Catalyst Example 1 may be referred to as "LBFMO (citric acid complex method)".

(Catalyst Example 2)

A dehydrogenating catalyst of Catalyst Example 2 was prepared by a solid state synthesis. Lanthanum oxide ($La_2O_3$), barium carbonate ($Ba(CO_3)_2$), iron oxide ($Fe_2O_3$), and manganese oxide ($MnO_2$) were mixed so that La, Ba, Fe, and Mn would have a molar ratio of 0.8:0.2:0.4:0.6 to obtain a powder mixture. The powder mixture was thoroughly pulverized and the particles of the powder mixture were thoroughly mixed with use of a wet-type bead mill to obtain pulverized, mixed powder. The pulverized, mixed powder was then dried to obtain a dried product. The dried product was fired under a condition in which the firing temperature was 1200°C and the temperature maintenance time was 5 hours. In this way, LBFMO was prepared. In the following description, the LBFMO of Catalyst Example 2 may be referred to as "LBFMO (solid state synthesis)".

(Catalyst Example 3)

The same operations as described in Catalyst Example 1 were carried out, except that lanthanum nitrate hexahydrate ($La(NO_3)_3 \cdot 6H_2O$), barium nitrate ($Ba(NO_3)_2$), and manganese nitrate hexahydrate ($Mn(NO_3)_3 \cdot 6H_2O$) were weighed out so that La, Ba, and Mn would have a molar ratio of 0.8:0.2:1 and used as a solute. In this way, $La_{0.8}Ba_{0.2}MnO_3$ (hereinafter may be referred to as "LBMO") was prepared.

(Catalyst Example 4)

The same operations as described in Catalyst Example 1 were carried out, except that lanthanum nitrate hexahydrate ($La(NO_3)_3 \cdot 6H_2O$), barium nitrate ($Ba(NO_3)_2$), and ferric nitrate nonahydrate ($Fe(NO_3)_3 \cdot 9H_2O$) were weighed out so that La, Ba, and Fe would have a molar ratio of 0.8:0.2:1 and used as a solute. In this way, $La_{0.8}Ba_{0.2}FeO_3$ (hereinafter may be referred to as "LBFO") was prepared.

(Catalyst Example 5)

The same operations as described in Catalyst Example 1 were carried out, except that cerium nitrate hexahydrate ($Ce(NO_3)_3 \cdot 6H_2O$) and manganese nitrate hexahydrate ($Mn(NO_3)_3 \cdot 6H_2O$) were weighed out so that Ce and Mn would have a molar ratio of 1: 1 and used as a solute. In this way, a mixture of $CeO_2$ and $Mn_2O_3$ at a molar ratio of 1:1 (hereinafter may be referred to as "CO+MO") was prepared.

(Catalyst Example 6)

The same operations as described in Catalyst Example 1 were carried out, except that cerium nitrate hexahydrate ($Ce(NO_3)_3 \cdot 6H_2O$) and manganese nitrate hexahydrate ($Mn(NO_3)_3 \cdot 6H_2O$) were weighed out so that Ce and Mn would have a molar ratio of 0.7:0.3 and used as a solute. In this way, $Ce_{0.7}Mn_{0.3}O_3$ (hereinafter may be referred to as "CMO (1)") was prepared. Note that two lots of the CMO (1) were prepared in Catalyst Example 6, which are referred to as CMO (1) (lot 1) and CMO (1) (lot 2), respectively.

(Catalyst Example 7)

The same operations as described in Catalyst Example 1 were carried out, except that cerium nitrate hexahydrate ($Ce(NO_3)_3 \cdot 6H_2O$) and manganese nitrate hexahydrate ($Mn(NO_3)_3 \cdot 6H_2O$) were weighed out so that Ce and Mn would have a molar ratio of 0.9:0.1 and used as a solute. In this way, $Ce_{0.9}Mn_{0.1}O_3$ (hereinafter may be referred to as "CMO (2)") was prepared.

(Comparative Example 2)

The same operations as described in Catalyst Example 1 were carried out, except that barium nitrate ($Ba(NO_3)_2$), zirconium oxynitrate dihydrate ($ZrO(NO_3)_2 \cdot 2H_2O$), and cerium nitrate hexahydrate ($Ce(NO_3)_3 \cdot 6H_2O$) were weighed out so that Ba, Zr, and Ce would have a molar ratio of 1:0.3:0.7 and used as a solute. In this way, $BaZr_{0.3}Ce_{0.7}O_3$ (hereinafter may be referred to as "BZCO") was prepared.

<Experiment of pyrolysis of ethane>

The following description will discuss an experiment of pyrolysis of ethane which was carried out with use of the LBFMO (citric acid complex method), LBFMO (solid state synthesis), LBMO, LBFO, CMO, and BZCO obtained by the foregoing methods. Note that, in Comparative Example 1, powdery $\alpha$-$Al_2O_3$ having no dehydrogenating catalyst supported thereon was used. The powdery $\alpha$-$Al_2O_3$ was produced by treating JRC-ALO-6 $\gamma$-$Al_2O_3$) (reference catalyst from the Catalysis Society of Japan) with heat under a condition in which temperature was 1300°C and the temperature maintenance time was 3 hours.

The experiment of pyrolysis of ethane was carried out in the following manner. First, a mixture of 100 mg of a sample (LBFMO (citric acid complex method), LBFMO (solid state synthesis), LBMO, LBFO, CMO, $\alpha$-$Al_2O_3$, or BZCO) and 392 mg of SiC which was an inert solid was filled into a quartz tube (having an inner diameter of 4 mm, a length of 180 mm) so that the height of the mixture in the quartz tube would be 30 mm. Next, the quartz tube was inserted into a tubular furnace, and the temperature in the quartz tube was raised to 700°C. Next, a gas was supplied to the quartz tube so as to cause a pyrolysis reaction of ethane in the quartz tube. The flow rates of raw materials were as follows: that is, ethane ($C_2H_6$): 18.1 mL/minute, moisture vapor ($H_2O$): 24.7 mL/minute, and $N_2$: 98.0 mL/minute. With regard to the gas flowing out of the quartz tube, hydrogen ($H_2$) and nitrogen ($N_2$) contained in the gas were analyzed with a TCG gas chromatograph (Shimadzu, GC-8A), and ethane ($C_2H_6$), ethylene ($C_2H_4$), carbon monoxide (CO), and methane ($C_2H_4$) contained in the gas were analyzed with an FID gas chromatograph (Shimadzu, GC-8A) provided with a methanizer to calculate the yield of ethylene ($C_2H_4$), the conversion ratio of ethane ($C_3H_6$), and selectivity of ethylene ($C_2H_4$).

The yield of ethylene for Catalyst Examples 1 to 7 and Comparative Examples 1 and 2 is shown in Table 1 and Fig. 4. (a) of Fig. 4 is a chart showing the yield of ethylene obtained in the experiment of pyrolysis of ethane which was carried out with use of dehydrogenating catalysts as Catalyst Examples and a Comparative Example and powdery $\alpha$-$Al_2O_3$. (b) of Fig. 4 is a chart showing selectivity of ethylene versus the conversion ratio of ethane obtained in the experiment of pyrolysis of ethane which was carried out with use of dehydrogenating catalysts as Catalyst Examples and a Comparative Example and powdery $\alpha$-$Al_2O_3$.

As shown in Table 1 and (a) of Fig. 4, the dehydrogenating catalysts of Catalyst Examples 1 to 7 were high in yield of ethylene than the dehydrogenating catalyst of Comparative Example 2 and the powdery $\alpha$-$Al_2O_3$ as Comparative Example 1. Furthermore, as shown in (b) of Fig. 4, the dehydrogenating catalysts of Catalyst Examples 1 to 7 were high in the conversion ratio of ethane than the dehydrogenating catalyst of Comparative Example 2 and the powdery $\alpha$-$Al_2O_3$ as Comparative Example 1.

Note that, in the experiment of pyrolysis of ethane, the dehydrogenating catalyst of each of Catalyst Examples 1 and 7 and Comparative Example 2 did not differ in color between before and after the reaction, and no coking was found. That is, it was confirmed that the dehydrogenating catalysts of Catalyst Examples 1 to 7 prevent coking and improve the yield of an olefin.

<X-ray diffraction analysis>

The dehydrogenating catalysts of Catalyst Examples 1 to 7 and Comparative Example 2 were subjected to an X-ray diffraction analysis. The crystallite size of each dehydrogenating catalyst was found from the results of the X-ray diffraction analysis. The crystallite sizes for Catalyst Examples 1 to 6 and Comparative Example 2 are shown in Table 1. (a) of Fig. 5 is a chart showing the yield of ethylene versus crystallite size obtained in the experiment of pyrolysis of ethane which was carried out with use of dehydrogenating catalysts as Catalyst Examples and a Comparative Example.

Note that the dehydrogenating catalyst of Catalyst Example 5 showed only peaks derived from $CeO_2$ and $Mn_2O_3$, which indicates that the dehydrogenating catalyst was a mixture of $CeO_2$ and $Mn_2O_3$ (physical mixture in which crystal structures exist independently of each other). It was found that, although $CeO_2$ and $Mn_2O_3$ each independently are low in catalytic performance, a mixture of them is higher in catalytic performance. Note that the crystallite size for Catalyst Example 5 shown in Table 1 is the crystallite size of $CeO_2$.

As shown in Table 1 and (a) of Fig. 5, the dehydrogenating catalysts of Catalyst Examples 1 to 6 had a crystallite size within the range of from 20 nm to 75 nm and achieved high yield.

The results of the X-ray diffraction analysis of the dehydrogenating catalysts of Catalyst Examples 1, 3, and 4 are shown in (a) of Fig. 6, and the results of the X-ray diffraction analysis of the dehydrogenating catalysts of Catalyst Examples 6 and 7 are shown in (b) of Fig. 6. The results of the X-ray diffraction analysis of the dehydrogenating catalyst of Comparative Example 2 are shown in (c) of Fig. 6.

It was confirmed that, with regard to the $La_{0.8}Ba_{0.2}Fe_{0.4}Mn_{0.6}O_3$ of Catalyst Example 1, a composite oxide represented by $La_{0.8}Ba_{0.2}Fe_{0.4}Mn_{0.6}O_3$ has been generated. It was confirmed that, with regard to the $La_{0.8}Ba_{0.2}MnO_3$ of Catalyst Example 3, a composite oxide represented by $La_{0.8}Ba_{0.2}MnO_3$ has been generated. It was confirmed that, with regard to the $La_{0.8}Ba_{0.2}FeO_3$ of Catalyst Example 4, a composite oxide represented by $La_{0.8}Ba_{0.2}FeO_3$ has been generated. The $Ce_{0.7}Mn_{0.3}O_3$ of Catalyst Example 6 (CMO (1) (lot 2)) showed only peaks derived from the reference sample $Ce_{0.7}Mn_{0.3}O_3$; therefore, it was confirmed that a composite oxide represented by $C_{0.7}M_{0.3}O_3$ has been generated. The $C_{0.9}M_{0.1}O_3$ of Catalyst Example 7 showed only peaks derived from the reference sample $Ce_{0.9}Mn_{0.1}O_3$; therefore, it was confirmed that a composite oxide represented by $C_{0.9}M_{0.1}O_3$ has been generated. The $BaZr_{0.3}Ce_{0.7}O_3$ of Comparative Example 2 showed only peaks derived from the reference sample $BaZr_{0.3}Ce_{0.7}O_3$; therefore, it was confirmed that a composite oxide represented by $BaZr_{0.3}Ce_{0.7}O_3$ has been generated. That is, the dehydrogenating catalysts of Catalyst Examples 1, 3, 4, 6, and 7 and Comparative Example 2 were each a perovskite-type oxide.

<Measurement of specific surface area>

The dehydrogenating catalysts of Catalyst Examples 1 to 6 and Comparative Example 2 were measured for specific surface area with use of a GeminiVII2390a (manufactured by Micromeritics). The specific surface areas for Catalyst Examples 1 to 6 and Comparative Example 2 are shown in Table 1. (b) of Fig. 5 is a chart showing the yield of ethylene versus specific surface area obtained in the experiment of pyrolysis of ethane which was carried out with use of dehydrogenating catalysts as Catalyst Examples and a Comparative Example.

As shown in Table 1 and (b) of Fig. 5, basically the yield was high and proportional to the specific surface area. Note that Catalyst Example 5 showed high yield and significantly deviated from a straight line.

[Table 1]

| | Sample | Crystallite size (nm) | Specific surface area (m²/g) | Yield of ethylene (mol%) |
|---|---|---|---|---|
| Comparative Example 1 | $\alpha$-$Al_2O_3$ | - | - | 0.95 |
| Comparative Example 2 | BZCO | 16.2 | 3.5 | 1.19 |
| Catalyst Example 1 | LBFMO (citric acid complex method) | 39.9 | 14 | 2.58 |
| Catalyst Example 2 | LBFMO (solid state synthesis) | 25 | 16.7 | 1.45 |
| Catalyst Example 3 | LBMO | 39.1 | 15.3 | 2.65 |
| Catalyst Example 4 | LBFO | 33.7 | 9.5 | 1.71 |
| Catalyst Example 5 | CO+MO | 37.2 ($CeO_2$) | 7.3 | 3.44 |
| Catalyst Example 6 | CMO (1) (lot 1) | - | - | 2.22 |
| | CMO (1) (lot 2) | 45.5 | 6.9 | 2.69 |
| Catalyst Example 7 | CMO (2) | - | - | 2.49 |

(ii) Second Working Example

The following description will discuss further Working Examples of a dehydrogenating catalyst for use in a pyrolysis tube for production of an olefin in accordance with an aspect of the present invention. The description in this section discusses Catalyst Example 1 as a Working Example of the dehydrogenating catalyst and Comparative Examples 3 and 4 as Comparative Examples.

<Method of producing dehydrogenating catalyst>

(Comparative Example 3)

A dehydrogenating catalyst of Comparative Example 3 was produced by applying an aqueous gallium nitrate $(Ga(NO_3)_2 \cdot nH_2O$, where n = 7 to 9) solution to $\alpha$-$Al_2O_3$ serving as a support and firing them at 1050°C for 3 hours in air. The dehydrogenating catalyst here was prepared so that the amount of gallium (Ga) would be 5% by weight the combined amount of gallium (Ga) and $\alpha$-$A1_2O_3$. The particle size of the fired dehydrogenating catalyst was adjusted to 350 mm to 500 mm. The sample obtained by the above method is hereinafter referred to as "Ga/$\alpha$-$Al_2O_3$".

(Comparative Example 4)

A dehydrogenating catalyst/promoter of Comparative Example 4 was produced by applying an aqueous solution of a mixture of gallium nitrate $(Ga(NO_3)_2 \cdot nH_2O$, where n = 7 to 9) and barium nitrate $(Ba(NO_3)_2)$ to $\alpha$-$Al_2O_3$ serving as a support and firing them at 1050°C for 3 hours in air. The dehydrogenating catalyst/promoter here was prepared so that the amount of gallium (Ga) would be 5% by weight the combined amount of gallium (Ga) and $\alpha$-$Al_2O_3$ and that the amount of barium (Ba) would be 0.1 times the amount of gallium (Ga) in terms of molar ratio. The particle size of the fired dehydrogenating catalyst/promoter was adjusted to 350 mm to 500 mm. The sample obtained by the above method is hereinafter referred to as "Ga-0.1Ba/$\alpha$-$Al_2O_3$".

<Experiment to evaluate the amount of carbon deposition>

A pyrolysis reaction of ethane was carried out in the same manner as described in First Working Example with use of the dehydrogenating catalysts of Catalyst Example 1 and Comparative Examples 3 and 4. After the pyrolysis reaction of ethane, with use of a thermal conductivity detector (TCD) (TGA-50 (manufactured by Shimadzu Corporation)), oxygen was passed through the dehydrogenating catalysts of Catalyst Example 1 and Comparative Examples 3 and 4 at a temperature falling within the range of from 100°C to 900°C, and carbon in the form of carbon monoxide (CO) and carbon dioxide $(CO_2)$ was detected. Fig. 7 is a chart showing the results of the experiment to evaluate the amount of carbon deposition which was carried out with use of the dehydrogenating catalysts as a Catalyst Example and Comparative Examples. The vertical axis "TCD signal" represents the amount of detected carbon monoxide and carbon dioxide.

With regard to the Ga/$\alpha$-$Al_2O_3$ of Comparative Example 3 and the Ga-0.1Ba/$\alpha$-$Al_2O_3$ of Comparative Example 4, carbon monoxide and carbon dioxide were detected. In contrast, with regard to the LBFMO of Catalyst Example 1, neither carbon monoxide nor carbon dioxide was detected. These results also show that the LBFMO of Catalyst Example 1 can prevent coking.

Industrial Applicability

The present invention is applicable to a pyrolysis tube for pyrolyzing a hydrocarbon raw material such as ethane or naphtha into an olefin.

Reference Signs List

 1A, 1A , 1B pyrolysis tube for production of olefin
 2 base material
 3 alumina layer (metal oxide layer)
 4A, 4B dehydrogenating catalyst
 4Ba catalyst component
 4Bb carrier
 5 plate-shaped member

**Claims**

1. A dehydrogenating catalyst for production of an olefin, comprising, as a catalyst component, at least one of the following composite oxide and the following mixture:

   a composite oxide that contains at least one of La and Ce, wherein, when the composite oxide does not contain Ce, the composite oxide contains at least one element selected from the group consisting of Ba, Fe, and Mn or wherein, when the composite oxide contains Ce, the composite oxide contains at least one of Fe and Mn and does not contain Ba; and
   a mixture that contains a first oxide and a second oxide, the first oxide containing at least one of La and Ce, wherein, when the first oxide does not contain Ce, the second oxide contains at least one element selected from the group consisting of Ba, Fe, and Mn or wherein, when the first oxide contains Ce, the second oxide contains at least one of Fe and Mn.

2. The dehydrogenating catalyst as set forth in claim 1, wherein the dehydrogenating catalyst has a specific surface area of 5 $m^2$/g to 80 $m^2$/g.

3. The dehydrogenating catalyst as set forth in claim 1 or 2, wherein the composite oxide or the first oxide has a crystallite size of 20 nm to 75 nm.

4. The dehydrogenating catalyst as set forth in any one of claims 1 to 3, wherein the composite oxide is a perovskite-type oxide.

5. A pyrolysis tube for production of an olefin, comprising:

   a tubular base material made of a heat resistant metal material and/or a plate-shaped member made of a heat resistant metal material; and
   a dehydrogenating catalyst as recited in any one of claims 1 and 4 supported on an inner surface of the tubular base material and/or a surface of the plate-shaped member.

6. A method of producing an olefin, comprising producing an olefin with use of a pyrolysis tube as recited in claim 5.

## FIG. 1

(a)

(b)

## FIG. 2

（a）

（b）

FIG. 3

（a）

（b）

# FIG. 4

FIG. 5

## FIG. 6

## FIG. 7

## INTERNATIONAL SEARCH REPORT

International application No.

PCT/JP2019/047229

### A. CLASSIFICATION OF SUBJECT MATTER
Int. Cl. B01J23/889(2006.01)i, B01J23/34(2006.01)i, B01J23/83(2006.01)i,
C07B61/00(2006.01)i, C07C5/333(2006.01)i, C07C11/04(2006.01)i
FI: B01J23/889 Z, B01J23/34 Z, B01J23/83 Z, C07B61/00 300, C07C5/333, C07C11/04
According to International Patent Classification (IPC) or to both national classification and IPC

### B. FIELDS SEARCHED

Minimum documentation searched (classification system followed by classification symbols)
Int. Cl. B01J23/889, B01J23/34, B01J23/83, C07B61/00, C07C5/333,
C07G11/04

Documentation searched other than minimum documentation to the extent that such documents are included in the fields searched
Published examined utility model applications of Japan      1922-1996
Published unexamined utility model applications of Japan    1971-2020
Registered utility model specifications of Japan            1996-2020
Published registered utility model applications of Japan    1994-2020

Electronic data base consulted during the international search (name of data base and, where practicable, search terms used)
CAplus/REGISTRY (STN)

### C. DOCUMENTS CONSIDERED TO BE RELEVANT

| Category* | Citation of document, with indication, where appropriate, of the relevant passages | Relevant to claim No. |
|---|---|---|
| X | WO 2010/032338 A1 (WASEDA UNIVERSITY) 25 March | 1-4 |
| Y | 2010, claims, paragraph [0020], examples 1, 2 | 5, 6 |
| X | JP 2017-521231 A (BASF SE) 03 August 2017, claims, | 1-4 |
| Y | catalysts K4-K8 | 5, 6 |
| X | BECKERS, Jurriaan et al., Selective Hydrogen | 1, 2, 4 |
| Y | Oxidation in the Presence of C3 Hydrocarbons Using Perovskite Oxygen Reservoirs, ChemPhysChem, 2008, vol. 9, pp. 1062-1068, 2.1, table 1, fig. 1 | 5, 6 |
| X | JP 2017-534623 A (SABIC GLOBAL TECHNOLOGIES BV) 24 | 1 |
| Y | November 2017, claims, paragraphs [0022], [0023], example 1 | 5, 6 |

☒ Further documents are listed in the continuation of Box C.  ☒ See patent family annex.

| | | | |
|---|---|---|---|
| * | Special categories of cited documents: | "T" | later document published after the international filing date or priority date and not in conflict with the application but cited to understand the principle or theory underlying the invention |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 03.02.2020 | 10.02.2020 |

| Name and mailing address of the ISA/ | Authorized officer |
|---|---|
| Japan Patent Office 3-4-3, Kasumigaseki, Chiyoda-ku, Tokyo 100-8915, Japan | Telephone No. |

Form PCT/ISA/210 (second sheet) (January 2015)

**INTERNATIONAL SEARCH REPORT**

| International application No. |
| --- |
| PCT/JP2019/047229 |

C (Continuation). DOCUMENTS CONSIDERED TO BE RELEVANT

| Category* | Citation of document, with indication, where appropriate, of the relevant passages | Relevant to claim No. |
| --- | --- | --- |
| Y | JP 2017-209661 A (KUBOTA CORP.) 30 November 2017, paragraphs [0083]-[0086], fig. 2 | 5, 6 |

Form PCT/ISA/210 (continuation of second sheet) (January 2015)

INTERNATIONAL SEARCH REPORT
Information on patent family members

| International application No. |
| --- |
| PCT/JP2019/047229 |

| Patent Documents referred to in the Report | Publication Date | Patent Family | Publication Date |
| --- | --- | --- | --- |
| WO 2010/032338 A1 | 25.03.2010 | US 2011/0178350 A1<br>claims, paragraph [0043], examples 1, 2<br>EP 2329878 A1<br>CN 102159312 A<br>KR 10-2011-0057184 A | |
| JP 2017-521231 A | 03.08.2017 | US 2017/0073284 A1<br>claims, catalysts K4-K8<br>WO 2015/169826 A1<br>EP 3140039 A1<br>CN 106413884 A | |
| JP 2017-534623 A | 24.11.2017 | US 2017/0313584 A1<br>claims, paragraphs [0023], [0024], example 1<br>WO 2016/069389 A1<br>EP 3212567 A1<br>CN 107406349 A | |
| JP 2017-209661 A | 30.11.2017 | US 2019/0309229 A1<br>paragraphs [0093]-[0096], fig. 2<br>WO 2017/199612 A1 | |

Form PCT/ISA/210 (patent family annex) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 9499747 B
- JP 2017209661 A